# FASCICULE DE BREVET EUROPEEN

(11) **EP 1 192 124 B1**
(45) Date de publication et mention de la délivrance du brevet: **11.08.2004**
(21) Numéro de dépôt: 01907646.2
(22) Date de dépôt: 19.01.2001
(51) Int. Cl.: C07C 219/08, C07C 213/08, C08F 20/34

(54) **PROCEDE DE FABRICATION DE MONOMERES A DEUX GROUPES AMINO QUATERNAIRES ET LES (CO)POLYMERES OBTENUS A PARTIR DE CES MONOMERES**
VERFAHREN ZUR HERSTELLUNG VON MONOMEREN MIT ZWEI QUATERNÄREN AMINOGRUPPEN UND (CO)POLYMEREN AUS DIESEN MONOMEREN
METHOD FOR MAKING MONOMERS WITH TWO QUATERNARY AMINO GROUPS AND (CO)POLYMERS OBTAINED FROM SAID MONOMERS

(30) Priorité: 24.01.2000 FR 0000834
(43) Date de publication de la demande: 03.04.2002
(73) Titulaire: Atofina, 92800 Puteaux (FR)
(72) Inventeur: RIONDEL, Alain, F-57600 Forbach (FR); TEMBOU, N'ZUDIE, Denis, F-27470 Serquigny (FR); ESCH, Marc, F-57800 Freyming Merlebach (FR); CHAPLINSKI, Vladimir, F-57500 Saint-Avold (FR); VANHOYE, Didier, F-60600 Breuil le Vert (FR)
(74) Mandataire: Chaillot, Geneviève
(86) Numéro de dépôt international: PCT/FR2001/000182
(87) Numéro de publication internationale: WO 2001/055089

(56) Documents cités:
- CZ-A- 250 962
- FR-A- 1 529 000
- SOLOVSKII, M. V. ET AL: "Synthesis and antimcrobial properties of mono- and polymeric quaternary ammonium salts containing aminoalkyl esters of methacrylic acid" KHIM.-FARM. ZH. (1974), 8(6), 20-4, 1974, XP000952593

## Description

La présente invention porte sur un procédé de fabrication de monomères à groupes amino tertiaires et/ou quaternaires.

Les composés du type de ceux de formule : 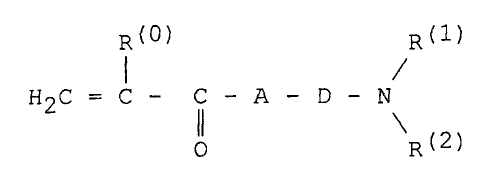 dans laquelle :
- R⁽⁰⁾ représente H ou CH₃ ;
- A représente -O- ou -NH- ;
- D représente une chaîne alkylène linéaire ou ramifiée en C₁-C₆ ;
- R⁽¹⁾ et R⁽²⁾, identiques ou différents, représentent chacun indépendamment H ou alkyle en C₁₋₅ ;
sont bien connus dans la littérature.

Des composés importants de cette famille sont l'acrylate de N,N-diméthylamino éthyle (ADAME) et le méthacrylate de N,N-diméthylamino éthyle (MADAME) : 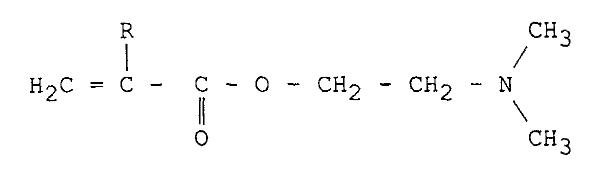 avec R = H ou CH₃.

Une très nombreuse littérature-brevets décrit la fabrication de solutions aqueuses de sels d'ammonium quaternaire ((M)ADAMQUAT), à partir de l'ADAME et du MADAME respectivement, ces sels, pour les plus représentatifs d'entre eux, pouvant être représentés par la formule : 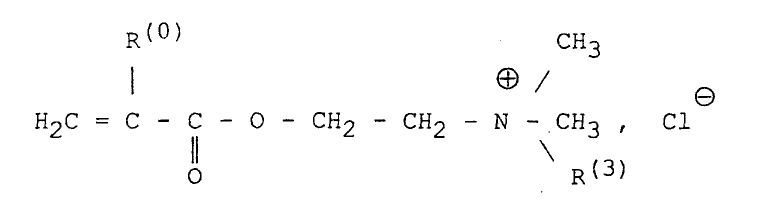 avec R = H ou -CH₃ et R⁽³⁾ = -CH₃ ou benzyle ((M)ADAMQUAT MC ou (M)ADAMAQUAT BZ suivant que R⁽³⁾ représente CH₃ ou benzyle).

Cette réaction est une quaternisation, en présence d'eau, du composé de départ avec un agent quaternisant R⁽³⁾ - Cl.

Les solutions aqueuses de sels quaternaires ainsi obtenues servent notamment à préparer des polymères destinés à servir de floculants cationigues dans le traitement des eaux.

SOLOVSKII, M.V. et al. "Synthesis and antimicrobial properties of mono- and polymeric quatemary ammonium salts containing aminoalkyl esters of methacrylic acid" KHIM.-FARM. ZH. (1974), 8(6), 20-4, 1974, décrit la préparation du 1,3-bis-(diméthyléthylammonium)isopropyl méthacrylate iodure, du 1,3-bis-(diméthylbenzylammonium)isopropyl méthacrylate iodure et du 1,3-bis(triméthylammonium)isopropyl méthacrylate méthosulfate en partant du mélange des réactifs, c'est-à-dire du méthacrylate de 1,3-bis(diméthylamino)isopropyl (DMAIM) avec respectivement l'iodure d'éthyle, l'iodure de benzyle et le diméthylsulfate.

Au cours de travaux de recherche et de développement sur ces polymères, la Société déposante a découvert que les composés de formule (I) : 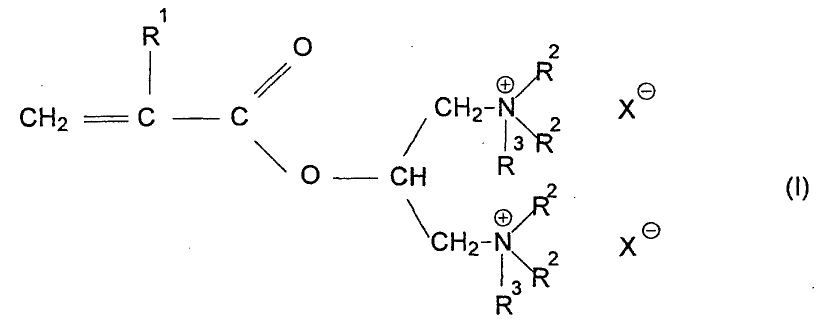 dans laquelle :
- R¹ représente H ou -CH₃ ;
- R² représente l'un parmi -CH₃, -C₂H₅, -C₃H₇ et -C₄H₉ ;
- les deux R³ sont identiques ou différents et représentent chacun l'un parmi -CH₃, -C₂H₅, -C₃H₇, -C₄H₉ et -CH₂C₆H₅ ; et
- les deux X^{⊖} sont identiques ou différents et représentent chacun Cl^{⊖} ou Br^{⊖},
connus par CZ-A-250 962, et en particulier ceux pour lesquels R² = CH₃, R³ = CH₃ ou benzyle et avec X^{⊖} = Cl^{⊖} (qui sont désignés ici par l'abréviation (M)ADAMQUAT 2MC ou 2BZ suivant que R³ représente CH₃ ou benzyle), permettent de préparer des dispersions aqueuses salines ou sans sel, apportant une solution à des problèmes techniques posés à l'homme du métier, ces dispersions faisant l'objet de trois demandes de brevets français déposées ce jour au nom de la Société déposante.

La présente invention a donc pour objet un procédé de fabrication de composé de la formule (I) telle que définie ci-dessus, caractérisé par le fait que l'on introduit au moins un agent quaternisant de formule (II) :

R³ - X^{⊖} (II)

dans laquelle R³ et X^{⊖} sont tels que définis ci-dessus, dans une solution, dans un solvant organique ou dans un mélange de solvants organiques, d'un composé de formule (III) : 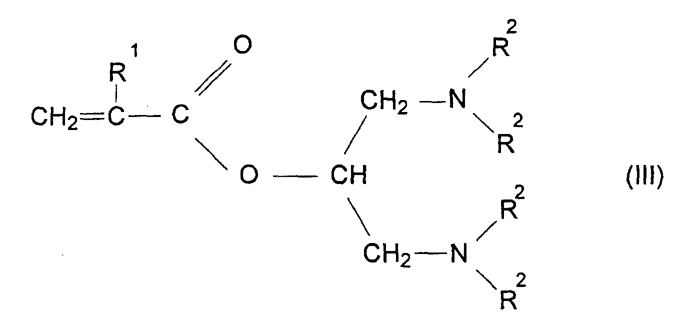 dans laquelle R¹ et R² sont tels que définis ci-dessus,
à une température de 35 à 80°C,
puis qu'on laisse se dérouler la réaction à ladite température jusqu'à disparition complète ou sensiblement complète du composé (III), et qu'on ajoute de l'eau, puis qu'on sépare une solution aqueuse de composé (I), et qu'on élimine l'eau le cas échéant.

A titre d'exemple de composé (I), on peut citer le composé de formule (Ia) : 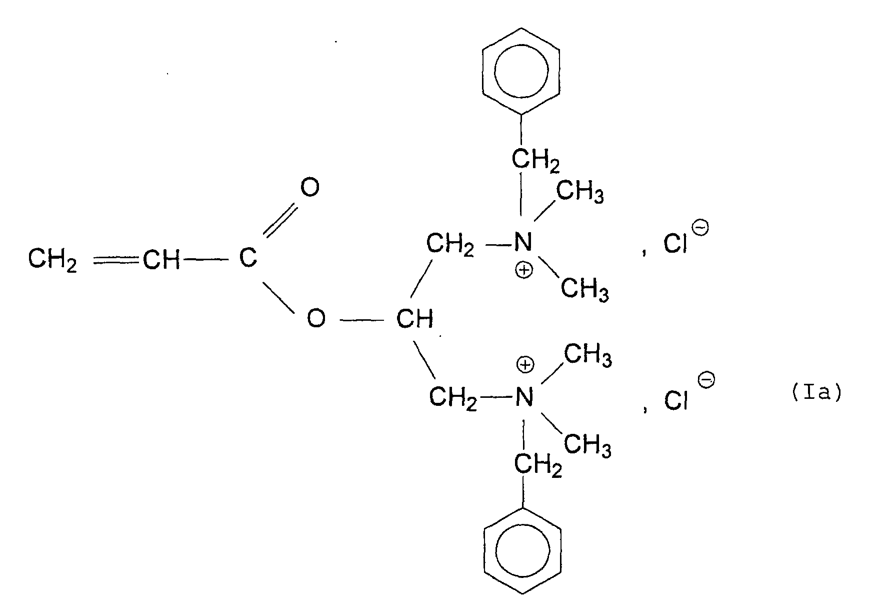 que l'on pourra désigner par l'abréviation S-ADAMQUAT 2BZ.

Avantageusement, on conduit la réaction avec un rapport molaire agent quaternisant (II) / composé (III) compris entre 1,8 et 3.

Le solvant organique utilisé est, par exemple, le chloroforme, le dichlorométhane ou le dichloroéthane. On peut également utiliser un mélange de tels solvants.

La réaction n'est généralement pas conduite sous pression excepté si l'agent quaternisant (II) est à l'état gazeux.

On introduit l'agent quaternisant (II) dans la solution du composé (III) généralement en l'espace de 0,5 -2 heures, et, après l'introduction de la totalité de l'agent quaternisant, on conduit la réaction des composés (II) et (III) généralement pendant un laps de temps de 10 à 40 heures.

Après la séparation de la solution aqueuse de composé (I), on préfère débarrasser la solution aqueuse obtenue de toute trace de solvant organique par stripping à l'air sous pression réduite.

Le procédé précité conduit à une solution aqueuse ayant une concentration en composé (I) qui est, de préférence, de 65 à 75% en poids.

Conformément à une caractéristique particulière du procédé ci-dessus, celui-ci est conduit en présence d'au moins un stabilisant choisi notamment parmi l'hydroquinone, l'éther méthylique de l'hydroquinone et le 3,5-ditert.-butyl-4-hydroxytoluène et les mélanges de ces stabilisants, la teneur en agent(s) stabilisant(s) étant notamment de 400 à 2000 ppm par rapport à la solution aqueuse de composé (I) final.

On peut préparer le composé (III) en faisant réagir un composé de formule (IV) : 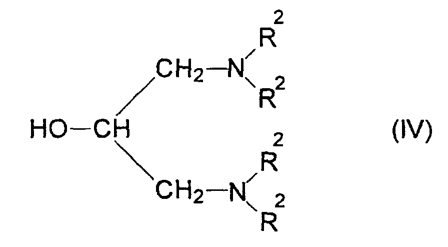 dans laquelle R² est tel que défini ci-dessus,
avec l'anhydride (méth)acrylique en présence de triéthylamine, avec un rapport molaire anhydride (méth)acrylique/composé (IV) de 0,5 à 2, à une température de 20 à 100°C, en particulier de 30 à 60°C, pendant un laps de temps de 2 à 10 heures, en présence d'au moins un stabilisant, tel que la phénothiazine, l'éther méthylique de l'hydroquinone, le 3,5-ditert.-butyl-4-hydroxytoluène et l'hydroquinone, et les mélanges de ces stabilisants, à raison de 200 à 3000 ppm par rapport à la charge.

Dans la réaction avec l'anhydride (méth)acrylique, la triéthylamine sert à catalyser la réaction et à piéger l'acide (méth)acrylique formé sous forme de sel. Elle est utilisée généralement à raison de 1 à 2 équivalents molaires par rapport à l'anhydride (méth)acrylique.

Les copolymères à base des monomères (I) incluant le monomère (Ia) peuvent être des polymères hydrosolubles ou hydrophobes ayant une présentation sous forme de dispersion aqueuse, latex, solution aqueuse, émulsion inverse ou de poudre. Ils sont préparés par copolymérisation radicalaire selon divers procédés de synthèse tels que les procédés de polymérisation en dispersion, solution, émulsion directe, émulsion inverse et suspension inverse.

Les exemples qui vont suivre, donnés à titre indicatif, permettent de mieux comprendre l'invention. Dans ces exemples, les parties et pourcentages indiqués sont en poids sauf indication contraire.

Dans ces exemples, les abréviations suivantes ont été utilisées :
S-ADAME : composé de formule : 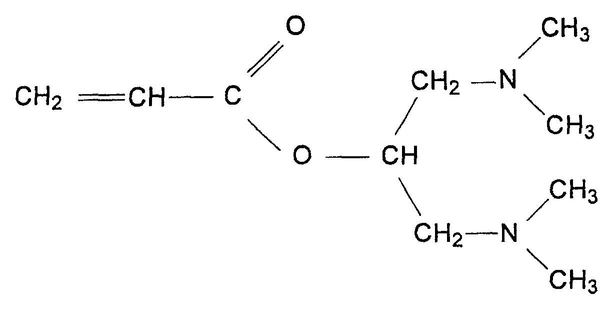
EMHQ : éther méthylique de l'hydroquinone.

### EXEMPLE 1 : Synthèse du S-ADAME

Dans un réacteur en verre de 1 litre, on charge :
- 292 g de 1,3-bis-diméthylamino-2 propanol ;
- 242 g de triéthylamine ; et
- 0,373 g de phénothiazine en tant que stabilisant.

Dans ce mélange agité, sous bullage d'air, à température ambiante, on ajoute, en 1 heure, 226 g d'anhydride acrylique. La température augmente pour atteindre 50°C. Après 2 heures supplémentaires de réaction, le mélange est refroidi et on ajoute 50 ml d'eau. Après décantation, on obtient une phase organique supérieure de 450 g, laquelle est distillée sous pression réduite pour isoler 250 g du composé de l'intitulé (pureté GC ≥ 99%).

### EXEMPLE 2 : Quaternisation du S-ADAME en S-ADAMOUAT 2BZ

Dans un réacteur en verre de 250 ml, on charge 44,2 g du S-ADAME obtenu au point (a) stabilisé avec 1500 ppm d'éther méthylique de l'hydroquinone et 150 g de CHCl₃. Le mélange sous agitation et sous bullage d'air est porté à 50°C. On ajoute en 1 heure, 55,9 g de chlorure de benzyle. Après 25 heures de réaction, l'acrylate de départ a disparu et l'on ajoute 33 g d'eau. On décante une phase supérieure qui est débarrassée des traces de CHCl₃ par stripping à l'air à 45°C sous pression réduite (P = 1,33 x 10⁴ Pa) (100 mm Hg)). On obtient ainsi 115,2 g de solution aqueuse contenant 75% de monomère cationique quaternaire ayant la structure attendue, déterminée par RMN ¹³C. Ce monomère est appelé S-ADAMQUAT 2BZ.

## Revendications

1. Procédé de fabrication de composé de formule (I) : 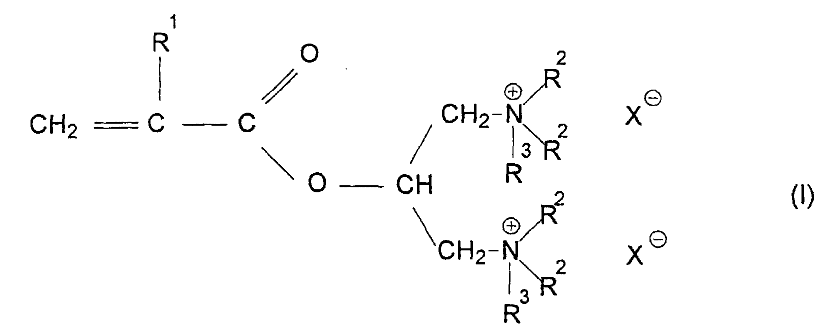 dans laquelle :
- R¹ représente H ou -CH₃ ;
- R² représente l'un parmi -CH₃, -C₂H₅, -C₃H₇ et -C₄H₉ ;
- les R³ sont identiques ou différents et représentent chacun l'un parmi -CH₃, -C₂H₅, -C₃H₇, -C₄H₉ et -CH₂C₆H₅ ; et
- les X^{⊖} sont identiques ou différents et représentent chacun Cl^{⊖} ou Br^{⊖},
**caractérisé par le fait que** l'on introduit au moins un agent quaternisant de formule (II) :
R³ - X^{⊖} (II)
dans laquelle R³ et X^{⊖} sont tels que définis ci-dessus, dans une solution, dans un solvant organique ou un mélange de solvants organiques, d'un composé de formule (III) : 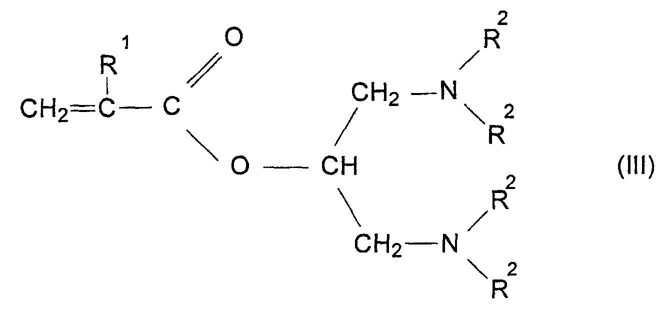 dans laquelle R¹ et R² sont tels que définis ci-dessus, à une température de 35 à 80°C,
puis qu'on laisse se dérouler la réaction à ladite température jusqu'à disparition complète ou sensiblement complète du composé (III), et qu'on ajoute de l'eau, puis qu'on sépare une solution aqueuse de composé (I), et qu'on élimine l'eau le cas échéant.

2. Procédé selon la revendication 1, **caractérisé par le fait que** l'on conduit la réaction avec un rapport molaire agent quaternisant (II) / composé (III) compris entre 1,8 et 3.

3. Procédé selon l'une des revendications 1 et 2, **caractérisé par le fait que** l'on utilise au moins l'un parmi le chloroforme, le dichlorométhane ou le dichloroéthane comme solvant organique.

4. Procédé selon l'une des revendications 1 à 3, **caractérisé par le fait qu'**il est conduit sous pression si l'agent quaternisant (II) est à l'état gazeux.

5. Procédé selon l'une des revendications 1 à 4, **caractérisé par le fait que** l'on introduit l'agent quaternisant (II) dans la solution du composé (III) en l'espace de 0,5 - 2 heures.

6. Procédé selon l'une des revendications 1 à 5, **caractérisé par le fait qu'**après l'introduction de la totalité de l'agent quaternisant, on conduit la réaction des composés (II) et (III) pendant un laps de temps de 10 à 40 heures.

7. Procédé selon l'une des revendications 1 à 6, **caractérisé par le fait que** l'on débarrasse la solution aqueuse obtenue de toute trace de solvant organique par stripping à l'air sous pression réduite.

8. Procédé selon l'une des revendications 1 à 7, **caractérisé par le fait qu'**il conduit à une solution aqueuse ayant une concentration en composé (I) de 65 à 75% en poids.

9. Procédé selon l'une des revendication 1 à 8, **caractérisé par le fait qu'**il est conduit en présence d'au moins un stabilisant choisi notamment parmi l'hydroquinone, l'éther méthylique de l'hydroquinone et le 3,5-ditert.-butyl-4-hydroxytoluène et les mélanges de ces stabilisants, la teneur en agent(s) stabilisant(s) étant notamment de 400 à 2000 ppm par rapport à la solution aqueuse de composé (I) final.

10. Procédé selon l'une des revendications 1 à 9, **caractérisé par le fait que** l'on prépare le composé (III) en faisant réagir un composé de formule (IV): 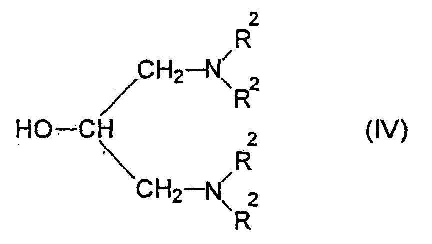 dans laquelle R² est tel que défini ci-dessus,
avec l'anhydride (méth)acrylique en présence de triéthylamine, avec un rapport molaire anhydride (méth)acrylique/composé (IV) de 0,5 à 2, à une température de 20 à 100°C, en particulier de 30 à 60°C, pendant un laps de temps de 2 à 10 heures, en présence d'au moins un stabilisant, tel que la phénothiazine, l'éther méthylique de l'hydroquinone, le 3,5-ditert.-butyl-4-hydroxytoluène et l'hydroquinone, et les mélanges de ces stabilisants, à raison de 200 à 3000 ppm par rapport à la charge.

## Patentansprüche

1. Verfahren zur Herstellung einer Verbindung der Formel (I): 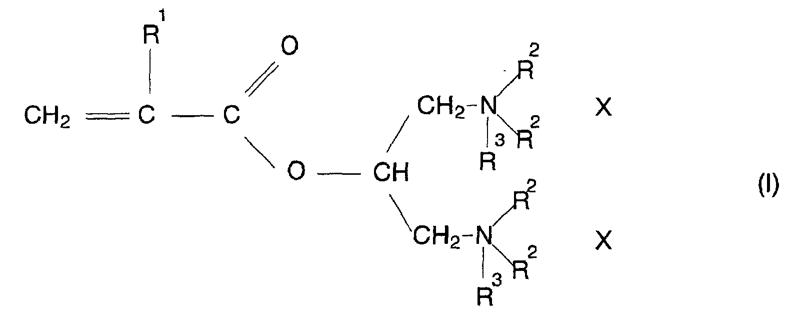 worin:
R¹ H oder -CH₃ darstellt;
R² einen von -CH₃, -C₂H₅, -C₃H₇ und -C₄H₉ darstellt;
die Reste R³ identisch oder verschieden sind und jeder von diesen -CH₃, -C₂H₅, C₃H₇, -C₄H₉ und -CH₂C₆H₅ darstellt; und
X^{⊖} identisch oder verschieden sind und jeweils Cl^{⊖} oder Br^{⊖} darstellen, **dadurch gekennzeichnet, daß** man wenigstens ein Quaternisierungsmittel der Formel (II):
R³-X^{⊖}, (II)
worin R³ und X^{⊖} wie oben definiert sind, in eine Lösung aus einem organischen Lösungsmittel oder einer Mischung organischer Lösungsmittel von einer Verbindung der Formel (III): 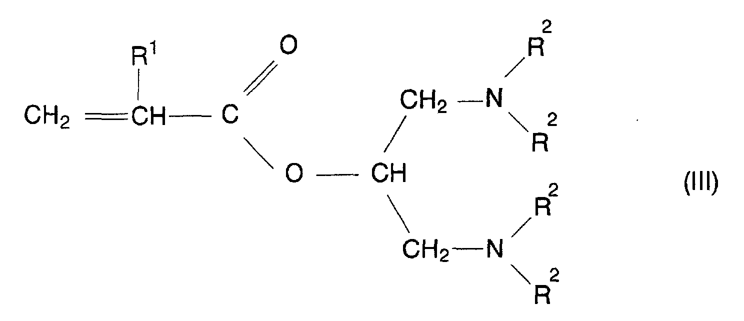 worin R¹ und R² wie oben definiert sind, bei einer Temperatur von 35 bis 80°C einführt, wobei man nachfolgend die Reaktion bei der genannten Temperatur bis zum vollständigen oder im wesentlichen vollständigen Verschwinden der Verbindung (III) ablaufen läßt und die Lösung in Wasser gibt, wobei man nachfolgend eine wässrige Lösung der Verbindung (I) abtrennt und das Wasser gegebenenfalls entfernt.

2. Verfahren gemäß Anspruch 1, **dadurch gekennzeichnet, daß** die Reaktion in einem Molverhältnis von Quarternisierungsmittel (II) / Verbindung (III) zwischen 1,8 und 3 durchführt.

3. Verfahren gemäß einem der Ansprüche 1 und 2, **dadurch gekennzeichnet, daß** man als organisches Lösungsmittel wenigstens eins von Chloroform, Dichlormethan oder Dichlorethan verwendet.

4. Verfahren gamäß einem der Ansprüche 1 bis 3, **dadurch gekennzeichnet, daß** es unter Druck durchgeführt wird, wenn das Quatemisierungsmittel (II) im Gaszustand vorliegt.

5. Verfahren gemäß einem der Ansprüche 1 bis 4, **dadurch gekennzeichnet, daß** das Quatemisierungsmittel (II) über einen Zeitraum von 0,5 bis 2 Stunden in die Lösung der Verbindung (III) gegeben wird.

6. Verfahren gemäß einem der Ansprüche 1 bis 5, **dadurch gekennzeichnet, daß** nach dem Zugeben der Gesamtmenge des Quarternisierungsmittels die Reaktion der Verbindungen (II) und (III) über einen Zeitraum von 10 bis 40 Stunden durchgeführ wird.

7. Verfahren gemäß einem der Ansprüche 1 bis 6, **dadurch gekennzeichnet, daß** die erhaltene wässrige Lösung von sämtlichen Spuren von organischen Lösungsmitteln durch Strippen unter vermindertem Druck befreit wird.

8. Verfahren gemäß einem der Ansprüche 1 bis 7, **dadurch gekennzeichnet, daß** es bis zu einer wässrigen Lösung mit einer Konzentration an Verbindung (I) von 65 bis 75 Gewichtsprozent durchgeführt wird.

9. Verfahren gemäß einem der Ansprüche 1 bis 8, **dadurch gekennzeichnet, daß** es in Gegenwart von wenigstens einem Stabilisator durchgeführt wird, gewählt insbesondere aus Hydrochinon, Hydrochinonmethylether und 3,5 di-tert.-Butyl-4-hydroxytoluol und den Mischungen dieser Stabilisatoren, wobei der Gehalt an Stabilisierungsmittel(n) insbesondere von 400 bis 2000 ppm in Bezug auf die wässrige Endlösung der Verbindung (I) beträgt.

10. Verfahren gemäß einem der Ansprüche 1 bis 9, **dadurch gekennzeichnet, daß** die Verbindung (III) durch Umsetzen einer Verbindung der Formel (IV): 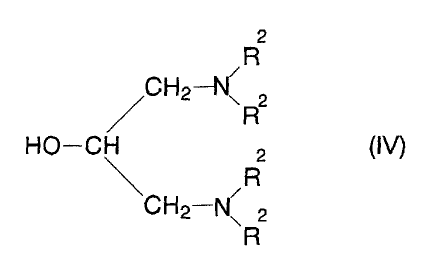 worin R² wie oben definiert ist, mit Methacrylsäureanhydrid in der Gegenwart von Triethylamin herstellt, mit einem Molverhältnis von Methacrylsäureanhydrid / Verbindung (IV) von 0,5 bis 2, bei einer Temperatur von 20 bis 100 °C, insbesondere von 30 bis 60°C, in einem Zeitraum von 2 bis 10 Stunden, in Gegenwart von wenigstens einem Stabilisator ausgewählt aus Phenothiazin, Hydrochinonmethylether, 3,5-di-tert.-Butyl-4-hydroxytoluol und Hydrochinon, und den Mischungen dieser Stabilisatoren, in einem Anteil von 200 bis 3000 ppm bezogen auf die Charge.

## Claims

1. Method of manufacturing a compound having formula (I): 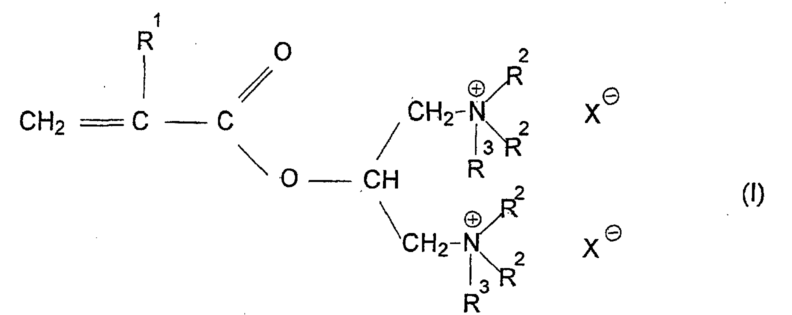 in which :
- R¹ represents H or -CH₃ ;
- R² represents one of -CH₃, -C₂H₅, -C₃H₇ and -C₄H₉ ;
- the two R³ are the same or different and each represents one of -CH₃, -C₂H₅, -C₃H₇, -C₄H₉ and -CH₂C₆H₅ ; and
- the two X^{⊖} are the same or different and each represents Cl^{⊖} or Br^{⊖},
**characterised in that** at least one quaternising agent of formula (II):
R³ - X^{⊖} (II)
in which R³ and X^{⊖} are as defined above,
is introduced into a solution, in an organic solvent or in a mixture of organic solvents, of a compound having formula (III): 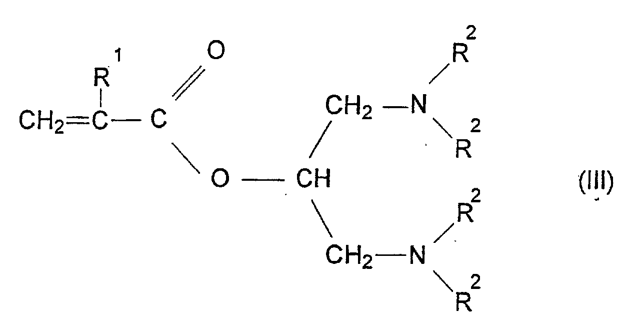 in which R¹ and R² are as defined above,
at a temperature of 35 to 80°C,
then the reaction is allowed to continue at said temperature until compound (III) has disappeared completely or substantially completely, after which water is added and then an aqueous solution of compound (I) is separated and the water removed as necessary.

2. Method as claimed in claim 1, **characterised in that** the reaction is conducted with a molar ratio of quaternising agent (II) / compound (III) in a range of between 1.8 and 3.

3. Method as claimed in one of claims 1 and 2, **characterised in that** the organic solvent used is at least one selected from chloroform, dichloromethane or dichloroethane.

4. Method as claimed in one of claims 1 to 3, **characterised in that** it is conducted under pressure if the quaternising agent (II) is in the gaseous state.

5. Method as claimed in one of claims 1 to 4, **characterised in that** the quaternising agent (II) is introduced into the solution of compound (III) over a period of 0.5 - 2 hours.

6. Method as claimed in one of claims 1 to 5, **characterised in that** once all of the quaternising agent has been introduced, the reaction between compounds (II) and (III) is conducted for a period of between 10 and 40 hours.

7. Method as claimed in one of claims 1 to 6, **characterised in that** all traces of organic solvent are removed from the resultant aqueous solution by stripping in air at a reduced pressure.

8. Method as claimed in one of claims 1 to 7, **characterised in that** it produces an aqueous solution with a concentration of 65 to 75% by weight of compound (I).

9. Method as claimed in one of claims 1 to 8, **characterised in that** it is conducted in the presence of at least one stabiliser chosen in particular from hydroquinone, hydroquinone methyl ether and 3,5-ditert.-butyl-4-hydroxytoluene and mixtures of these stabilisers, the content of stabilising agent(s) being in particular from 400 to 2000 ppm relative to the aqueous solution of the final compound (I).

10. Method as claimed in one of claims 1 to 9, **characterised in that** compound (III) is prepared by causing a compound of formula (IV): 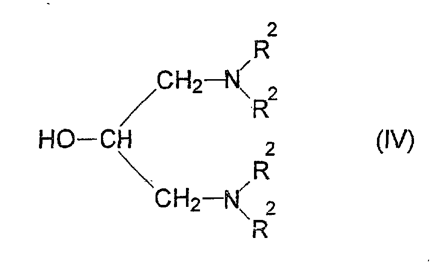 in which R² is as defined above,
to react with (meth)acrylic anhydride in the presence of triethylamine, with a molar ratio of (meth)acrylic anhydride / compound (IV) of 0.5 to 2, at a temperature of 20 to 100°C, in particular 30 to 60°C, for a period of 2 to 10 hours in the presence of at least one stabiliser such as phenothiazine, hydroquinone methyl ether, 3,5-ditert.-butyl-4-hydroxytoluene and hydroquinone and mixtures of these stabilisers in a ratio of 200 to 3000 ppm relative to the charge.
